# EUROPEAN PATENT SPECIFICATION

(11) **EP 3 316 859 B1**
(45) Date of publication and mention of the grant of the patent: **11.03.2020**
(21) Application number: 16734332.6
(22) Date of filing: 30.06.2016
(51) Int. Cl.: A61K 9/107, A61K 31/05, A61P 25/20

(54) **PROPOFOL EMULSION FOR PARENTERAL ADMINISTRATION**
PROPOFOLEMULSION ZUR PARENTERALEN VERABREICHUNG
ÉMULSION DE PROPOFOL POUR ADMINISTRATION PARENTÉRALE

(30) Priority: 01.07.2015 EP 15174744
(43) Date of publication of application: 09.05.2018
(73) Proprietor: Fresenius Kabi Deutschland GmbH, 61352 Bad Homburg (DE)
(72) Inventor: BRITO DE LA FUENTE, Edmundo, 61381 Friedrichsdorf (DE); GALLEGOS-MONTES, Crispulo, 61352 Bad Homburg (DE); HEKMATARA, Telli, 60316 Frankfurt am Main (DE); QUINCHIA-BUSTAMENTE, Lida A., 61352 Bad Homburg (DE)
(74) Representative: Fresenius Kabi Deutschland GmbH
(86) International application number: PCT/EP2016/065279
(87) International publication number: WO 2017/001549

(56) References cited:
- WO-A1-2016/126930
- WO-A2-2007/052288
- US-A1- 2004 225 022
- WEIHUI CAI ET AL: "A propofol microemulsion with low free propofol in the aqueous phase: Formulation, physicochemical characterization, stability and pharmacokinetics", INTERNATIONAL JOURNAL OF PHARMACEUTICS, vol. 436, no. 1-2, 1 October 2012 (2012-10-01), pages 536-544, XP055208261, ISSN: 0378-5173, DOI: 10.1016/j.ijpharm.2012.07.008

## Description

### Field of the disclosure

The present disclosure relates to compositions for parenteral administration comprising propofol.

The present disclosure further relates to a method for manufacturing the compositions of the disclosure as well as to the use of the compositions of the disclosure.

### Background

Propofol (2,6-diisopropylphenol) is a short acting anaesthetic that is registered for the induction and maintenance of anaesthesia in adults and children above 3 years of age, for sedation during surgical and diagnostic measures and for sedation of ventilated patients above 16 years of age in the context of intensive care.

Propofol offers various advantages over other agents, such as rapid onset, short duration of action, and excellent quality of recovery. Propofol is a highly lipophilic liquid, its lipophilicity allowing for easy permeation of the blood brain barrier, resulting in a rapid anaesthetic effect. Furthermore, owing to its high clearance rate and a short elimination half-life propofol has a low tendency to accumulate in the body.

However, due to the poor water solubility of propofol, the development of injectable formulations has been a particular challenge.

The first clinical trials have been conducted with a formulation containing Cremophor EL, a polyethoxylated castor oil. This formulation has been associated with the incidence of anaphylaxis.

Today, propofol is marketed in form of oil-in-water emulsions, e.g. under the trade name Diprivan® by Astra Zeneca.

Despite the success of the currently available products, several drawbacks have been reported. These include instability of the emulsion, induction of hyperlipidemia and/or liver disease, rapid growth of microorganisms as well as pain upon injection. The latter has been associated with the free propofol concentration in the aqueous phase (see e.g. Hiroshi Ohmio et al., Can J Anesth 2005, 52:6, pp 595-599 and WeiHui Cai et al., International Journal of Pharmaceutics 2012, 436, pp 536-544). Accordingly, low concentrations of free propofol in the aqueous phase are desirable.

Alternative approaches, such as for example the formation of microemulsions, the use of prodrugs or the formation of complexes with cyclodextrins, have only been partly successful as they are associated with disadvantages of their own.

The formation of cyclodextrin complexes is not an option for alpha- and beta-cyclodextrins as these show renal toxicity and are thus not suitable for parenteral application at all. Other cyclodextrins may only be administered in limited amounts (see "Background review for cyclodextrins used as excipients" issued by the EMEA on 20 November 2014, downloaded on 18 June 2015 from the homepage of the EMEA: http://www.ema.europa.eu/docs/en GB/document library/Report/2014/12/WC500177936.pdf).

Microemulsions require the addition of large amounts of synthetic emulsifiers being less compatible than naturally occurring lecithins.

The use of prodrugs of propofol instead of propofol itself is hampered by the fact that pharmacokinetics are changed.

### Summary

It has now surprisingly been found that the above mentioned drawbacks may be overcome by the compositions of the present disclosure.

Specifically, it has surprisingly been found that propofol may be provided in form of an emulsion comprising low concentrations of free propofol in the aqueous phase.

Thus, the present disclosure relates to an emulsion comprising 0.1 to 10 wt.%, preferably 1 to 5 wt.%, propofol and 5 to 25 wt.%, preferably 10 to 20 wt.%, of an oil phase based on the total weight of the emulsion, wherein the free propofol concentration in the aqueous phase is below 0.1 % of the total propofol content and wherein the oil phase comprises fish oil, fish oil extract or a mixture of fish oil, olive oil, soybean oil and medium chain triglycerides (MCT).

Particularly preferred embodiments are set forth in the claims.

### Detailed description

### Free propofol concentration in the aqueous phase

The term "free propofol concentration in the aqueous phase" as used in the present disclosure refers to the concentration of free propofol in the aqueous phase as measured by the following method:
Propofol emulsions (aliquots of 30 ml) were dialyzed for 24 hours at 25 °C using a dialysis membrane with a cutoff molecular weight of 3500 Da (dialysis tube: Roth No. E656.1) versus an aqueous solution of 2.5 % glycerol.

The concentrations of free propofol in the aqueous phase were measured by high performance liquid chromatography (e.g. using the Agilent Technologies 1200 series), using a mixture of water and acetonitrile as mobile phase at the following gradient:

| Time [min] | water | acetonitrile |
|---|---|---|
| 0.0 | 35 | 65 |
| 2.5 | 35 | 65 |
| 2.6 | 10 | 90 |
| 4.6 | 10 | 90 |

The flow rate was 1.0 ml/min, the injection volume was 10 µl, and the components of the column effluent were monitored using an ultraviolet detector at a wavelength of 275 nm. Calculations were performed by means of an external calibration with the analyte using 3-point-calibration.

### Emulsion

The compositions according to the present disclosure are emulsions, preferably oil-in-water emulsions, i.e. the continous phase is aqueous and comprises oil droplets. The emulsion comprises the continous aqueous phase and preferably 5 wt.% to 25 wt.% of an oil phase based on the total weight of the emulsion. More preferably, the emulsion comprises 10 wt% to 20 wt.% of an oil phase based on the total weight of the emulsion. For example, the emulsion comprises 10 wt.% or 20 wt.% of an oil phase based on the total weight of the emulsion.

The aqueous phase comprises water in purity suitable for parenteral administration, i.e. water for injection.

Oil-in-water emulsions for parenteral administration have to be sterile, pyrogen-free, well tolerated, isotonic or as close as possible to isotonicity, free of particulate impurities and storage stable. Their pH should be as close as possible to the pH of the blood.

Because fat globules larger than 5 µm may induce occlusion of the microvasculature, oil-in-water emulsions for parenteral administration must contain only very few oil droplets larger than 5 µm.

The USP refers to this parameter as "PFAT₅", which should not exceed 0.05 (compare USP 36 NF31 <729>).

Scientifically correct, the PFAT₅ value refers to the volume-weighted, large-diameter fat globule limits of the dispersed phase, expressed as the percentage of fat residing in globules larger than 5 µm (PFAT₅).

### The oil phase

The oil phase may comprise a variety of different lipids.

The oil phase comprises fish oil, fish oil extract or a mixture of fish oil, olive oil, soybean oil and medium chain triglycerides (MCT).

The oil phase may further comprise additional lipids, e.g. oils, e.g. safflower oil, corn oil, sunflower oil, coconut oil, palm kern oil, rapseed oil and mixtures thereof.

The term "fish oil" refers to "purified fish oil" and to "purified fish oil rich in omega 3 fatty acids", the latter according to the European Pharmacopoeia 6.0 comprising at least 9 wt.% of the omega-3-fatty acid docosahexaenoic acid (DHA) and at least 13 wt.% of the omega-3 fatty acid eisosapentaenoic acid (EPA) expressed as triglycerides.

The term "fish oil extract" refers to mixtures highly concentrated in EPA and DHA obtained e.g. from fish oil e.g. by supercritical fluid extraction and subsequent purification via e.g. chromatographic methods. Alternatively, the oil can be extracted using extraction techniques such as the one described in US6750048. Additional extraction and/or purification techniques are described in WO2001/076715 and WO2001/076385. The sum of EPA and DHA contained in these fish oil extracts is at least 500 milligram per gram of extract.

The fish oil extract comprises EPA and DHA in esterified form, e.g. in form of triglycerides or ethyl esters.

The term "medium chain triglycerides" refers to triglycerides of fatty acid being 6 to 12 carbon atoms in length, including caproic acid, caprylic acid, capric acid and lauric acid.

### The emulsifier

The emulsions according to the present disclosure comprise at least one pharmaceutically acceptable emulsifier. The term "emulsifier" refers to compounds which stabilize the composition by reducing the interfacial tension between the oil phase and the water phase and which typically comprise at least one hydrophobic group and at least one hydrophilic group. These emulsifiers (which may also be referred to as surfactants) are preferably used in amounts effective to provide, optionally together with further surfactants present, a stable and even distribution of the oil phase within the aqueous phase. In particular, the emulsifier is selected from the group of emulsifiers that have been approved for parenteral administration.

Preferably, the at least one emulsifier is lecithin. Within the meaning of the present disclosure the term "lecithin" refers to naturally occurring or synthetic lecithin that may be suitably refined. Suitable lecithins include, but are not limited to, lecithins derived from egg, corn or soybean or mixtures thereof. Lecithins are typically mixtures of diglycerides of fatty acids linked to the choline ester of phosphoric acid and can contain differing amounts of other compounds depending on the method of isolation. Typically, commercial lecithin is a mixture of acetone-insoluble phosphatides. Preferably, the lecithin is obtained from egg or from seeds including soybean and corn, using methods well known in the art. Lecithin obtained from soybean is referred to herein as soy lecithin. Lecithin obtained from egg is referred to herein as egg lecithin.

Preferably, the emulsions comprise lecithin as emulsifier, more preferably the lecithin is selected from the group consisting of egg lecithin, soy lecithin, and mixtures thereof.

These are commercially available, e.g. under the trade names Epikurin™170 (soy lecithin), PL 90 or Lipoid E80 (both egg lecithin).

Preferably, the lecithin is used in an amount of 0.5 to 5 wt.%, more preferably 0.5 to 3 wt.%, most preferably 1.0 to 2.5 wt.%.

Alternatively, krill oil may be used as an emulsifier. Krill oil is an extract prepared from a species of antarctic krill, Euphausia superba. It has obtained GRAS (generally recognized as safe) status from the FDA and is commercially available, e.g. from Olympic Seafood (Bioriginal Europe/Asia B.V.) and Aker BioMarine Antarctic AS. The emulsifying properties of krill oil mainly rely on its content in phospholipids (including phosphatidylcholine, phosphatidylethanolamine and phosphatidylinositol).

### The antioxidant

The emulsion may comprise at least one pharmaceutically acceptable antioxidant.

An antioxidant useful in the emulsion of the disclosure may be any pharmaceutically acceptable compound having antioxidant activity, for example, the antioxidant may be selected form the group consisting of sodium metasulfite, sodium bisulfite, sodium sulfite, sodium thiosulfate, thioglycerol, thiosorbitol, thioglycolic acid, cysteine hydrochloride, n-acetly-cysteine, citric acid, alpha-tocopherol, beta-tocopherol, gamma-tocopherol, soluble forms of vitamin E, butylated hydroxyanisole (BHA), butylated hydroxytoluene (BHT), t-butylhydroquinone (TBHQ), monothioglycerol, propyl gallate, histidine, enzymes such as superoxide dismutase, catalase, selenium glutathione peroxidase, phospholipid hydroperoxide and glutathione peroxidase, Coenzyme Q10, tocotrienols, carotenoids, quinones, bioflavonoids, polyphenols, bilirubin, ascorbic acid, isoascorbic acid, uric acid, metal-binding proteins, ascorbic acid palmitate, an antioxidant obtained or obtainable from rosemary, rosemary extract and mixtures thereof.

The at least one antioxidant is in particular selected from the group consisting of alpha-tocopherol, beta-tocopherol, gamma-tocopherol, ascorbic acid, and mixtures of two or more thereof.

If present, the total amount of agents with antioxidant activity is preferably in the range of from 0.01 wt.% to 0.05 wt %, more preferably from 0.01 wt.% to 0.04 wt.%, more preferably from 0.01 wt.% to 0.03 wt.%, and even more preferably from 0.015 wt.% to 0.025 wt.% based on the total weight of the emulsion.

### The tonicity agent

The emulsion may comprise at least one pharmaceutically acceptable tonicity agent.

Tonicity agents are used to confer tonicity. Suitable tonicity agents may be selected from the group consisting of sodium chloride, mannitol, lactose, dextrose, sorbitol and glycerol.

Preferably, the tonicity agent is glycerol.

Preferably, the total amount of tonicity agents is in the range of 0.1 to 10 wt.%, more preferably from 1 wt.% to 5 wt.%, more preferably from 1 wt.% to 4 wt.%, more preferably 1 wt.% to 3 wt.%, more preferably from 1.5 wt.% to 2.8 wt.%, and even more preferably from 2.0 wt.% to 2.8 wt.% based on the total weight of the emulsion.

In case the tonicity agent is glycerol the most preferred amount is 2.0 wt.% to 2.5 wt.% based on the total weight of the emulsion.

Preferably, the emulsion has an osmolality in the range of 305 to 420 mOsmol/kg, measured with a Vapor Pressure Osmometer, Model 5520 (Vapro TM) according to USP <785>.

### pH adjustment

The pH of the emulsion may be adjusted by adding solutions of conventionally known acids or bases such as HCI and NaOH or through the use of buffers, such as phosphate buffers.

The final pH of the emulsion is preferably in the range of from 6 to 9, more preferably between 7 and 9.

Preferably, the pH of the emulsion according to the disclosure is adjusted using a solution of NaOH.

### The co-surfactant

The emulsion according to the disclosure may further comprise a pharmaceutically acceptable co-surfactant.

A co-surfactant is an amphiphilic molecule, i.e. a molecule that contains both hydrophilic and lipophilic groups. Usually, a co-surfactant substantially accumulates with the emulsifier at the interfacial layer. The hydrophile-lipophile balance (HLB) number is used as a measure of the ratio of hydrophilic and lipophilic groups present in a surfactant or co-surfactant, respectively. Usually, a co-surfactant with a very low HLB value (thus with a relatively high affinity to oil) is used together with a surfactant with a high HLB to modify the overall HLB of the system. Unlike the emulsifier, the co-surfactant may not be capable of forming self-associated structures, like micelles, on its own. Several kinds of molecules including nonionic emulsifiers, alcohols, amines and acids, can function as co-surfactants in a given system. The co-surfactant is usually used in a lower amount than that of the emulsifier. Apart from modifying the overall HLB value of the system, the co-surfactant has the effect of further reducing the interfacial tension and increasing the fluidity of the interface. Co-surfactants may also adjust the curvature of the interfacial film by partitioning between the tails of the emulsifier chains, allowing greater penetration of the oil between the emulsifier tails.

Preferably, the co-surfactant is a free unsaturated fatty acid or a salt thereof, preferably an omega-9 fatty acid or a salt thereof, more preferably a monounsaturated omega-9 fatty acid or a salt thereof, more preferably oleic acid or sodium oleate.

The total amount of the co-surfactant is preferably in the range of from 0.01 wt.% to 1 wt.%, more preferably in the range of from 0.02 wt.% to 0.5 wt.%, more preferably in the range of from 0.02 wt.% to 0.20 wt.% based on the total weight of the emulsion.

### The co-solvent

The emulsion according to the disclosure may further comprise a pharmaceutically acceptable co-solvent.

The term co-solvent refers to molecules that may increase the stability of the emulsion. In addition to making the environment more hydrophobic by reducing the dieelectric constant of water, co-solvents increase the amount of molecularly dispersed emulsifier and/or co-surfactant in the aqueous phase. Availability of free surfactant aids in the solubilization of hydrophobic molecules by creating pockets of hydrophobic regions within the aqueous phase.

The co-solvent may be selected from the group consisting of ethanol, propylene glycol and polyethylen glycol.

Preferably, the co-solvent is a polyalkylene glycol or an alkylene glycol, preferably polyethylen glycol or polypropylen glycol, more preferably polyethylene glycol (PEG).

The PEG preferably has a mean molecular weight in the range of from 100 to 20000 Da, more preferably in the range of from 200 to 1000 Da, more preferably in the range of from 300 to 600 Da, most preferably around 400 Da.

Preferably, the co-solvent is selected from the group consisting of PEG 200, PEG 300, PEG 400, PEG 600, PEG, 1000, PEG 1450, PEG 4000, PEG 6000, PEG 8000 and PEG 20000. Most preferably, the co-solvent is PEG 400.

Preferably, the total amount of co-solvents ranges from 0.1 wt% to 2.0 wt.%, more preferably from 0.25 wt.% to 1.75 wt.%, more preferably from 0.50 wt.% to 1.50 wt.%, more preferably from 0.70 wt.% to 1.40 wt.%, more preferably from 0.80 wt.% to 1.30 wt.%, and even more preferably from 0.90 wt.% to 1.20 wt.% based on the total weight of the emulsion.

### The droplet size

Since the emulsion of the disclosure is an oil-in-water emulsion, the continous phase is aqueous and comprises oil droplets. These oil droplets are stabilized within the aqueous phase by at least one emulsifier and optionally further additives. The size of the oil droplets depends on the qualitative and quantitative composition of the emulsion and its preparation.

The oil droplets of the emulsion herein preferably have a mean diameter of 130 to 300 nm when measured directly upon sterilization using an LS 13 320 Laser Diffraction Particle Size Analyser (Beckman Coulter) according to USP <729>.

### Preparation of the emulsion

The present disclosure also relates to a method for preparing an emulsion for parenteral administration and to an emulsion obtained or obtainable by said method, wherein the method comprises
a) providing an oil phase comprising fish oil, fish oil exract or a mixture of fish oil, olive oil, soybean oil and MCT, propofol and optionally at least one pharmaceutically acceptable antioxidant and/or a pharmaceutically acceptable co-surfactant,
b) providing an aqueous phase comprising water for injection and optionally a pharmaceutically acceptable tonicity agent and/or an agent for pH adjustment and/or a pharmaceutically acceptable co-surfactant and/or a pharmaceutically acceptable co-solvent,
c) forming a pre-emulsion by mixing the oil phase provided in step a) with the aqueous phase provided in step b);
d) forming the emulsion by high-pressure homogenizing the pre-emulsion obtained in step c) and
e) sterilizing the emulsion obtained in step d),
wherein optionally either in step a) or in step b) a pharmaceutically acceptable emulsifier is added.

It is to be understood that any of the optional further components of the emulsion may be added in any of the steps a), b), c) or d) or in one or more additional steps.

### Step a)

Step a) is preferably carried out by mixing one or more lipids as defined in claim 1, propofol and optionally a pharmaceutically acceptable antioxidant and/or co-surfactant. This step is preferably carried out at a temperature of 55 to 65 °C, wherein during this step the temperature may be varied or held essentially constant for a maximum 30 minutes until a homogeneous and clear phase is obtained.

It is to be understood that in step a) further additives may be added.

In particular, it is to be understood that the at least one pharmaceutically acceptable emulsifier - depending on its nature - may be added either in step a) or in step b).

### Step b)

Step b) is preferably carried providing water for injection and optionally adding a pharmaceutically acceptable tonicity agent and/or co-surfactant.

The aqueous phase is then heated to a temperature of 55 to 65 °C, preferably for a time of 1 minute to 1 hour, more preferably from 5 to 30 minutes, more preferably from 5 to 15 minutes.

Preferably, step b) further comprises adjusting the pH to values between 7 and 10, preferably to a pH between 8 and 9, preferably by adding a solution of NaOH.

It is to be understood that in step b) further additives may be added.

In particular, it is to be understood that the at least one pharmaceutically acceptable emulsifier - depending on its nature - may be added either in step a) or in step b).

### Step c)

The method further comprises mixing the oil phase provided in step a) with the aqueous phase provided in step b) thereby forming a pre-emulsion. The mixing may be carried out by any method known to those skilled in the art. Preferably, the mixing is carried out using a high shear mixer.

Preferably the oil phase is added to the aqueous phase or vice-versa at a temperature in the range of from 55 to 65 °C.

Preferably the oil phase is added to the aqueous phase or vice-versa at a pressure such as as under nitrogen pressure, in the range of from 0.20 to 0.80 bar, more preferably from 0.2 to 0.4 bar. During this step the pressure may be varied or held essentially constant.

According to a preferred embodiment, the mixture is stirred for a time in the range of from 1 minute to 1 hour, preferably from 10 to 30 minutes. During this step, the temperature may be varied or held essentially constant.

It is to be understood that further components may also be added after the formation of the pre-emulsion. According to a preferred embodiment, the pH of the pre-emulsion is adjusted to a pH in the range of from 8 to 10, in particular by adding sodium hydroxide, if necessary.

### Step d)

The method further comprises the homogenization of the pre-emulsion obtained in step c). This homogenization may be carried out by any suitable method known to those skilled in the art.

Preferably the mixture is homogenized at a temperature in the range of from 40 to 60 °C, preferably from 45 to 55 °C.

Preferably, the pre-emulsion is homogenized at a pressure in the range of from 400 to 600 bar, more preferably from 450 to 550 bar. During this step the pressure may be varied or held essentially constant.

Preferably, the homogenization is carried out using a high pressure homogenizer or a microfluidizer.

### Step e)

The method further comprises the sterilization of the emulsion obtained in step d) to ensure its suitability for parenteral administration.

The sterilization may be carried out by any suitable method known to those skilled in the art. In particular, the sterilization is carried out by autoclaving, preferably at a temperature in the range of from 119 to 122 °C, more preferably at a temperature around 121 °C, preferably for a time in the range of from 1 minute to 30 minutes, preferably of from 10 minutes to 15 minutes.

### Route of administration

The compositions according to the present disclosure are adapted for parenteral administration, i.e. for a route of administration "other than via the gastrointestinal tract". Preferably, the compositions according to the present disclosure are administered intravenously.

The present disclosure includes inter alia the following aspects:
In a first aspect the present disclosure relates to an emulsion for parenteral administration comprising 0.1 to 10 wt.%, preferably 1 to 5 wt.%, propofol and 5 to 25 wt.%, preferably 10 to 20 wt.%, of an oil phase based on the total weight of the emulsion, wherein the free propofol concentration in the aqueous phase is below 0.1 % of the total propofol content and wherein the oil phase comprises fish oil, fish oil extract or a mixture of fish oil, olive oil, soybean oil and medium chain triglycerides (MCT).

In a second aspect the present disclosure relates to an emulsion according to aspect 1 comprising a pharmaceutically acceptable emulsifier, preferably egg lecithin or krill oil.

In a third aspect the present disclosure relates to an emulsion according to aspect 1 or 2 comprising a pharmaceutically acceptable tonicity agent, preferably glycerol.

In a fourth aspect the present disclosure relates to an emulsion according to any of the preceding aspects comprising a pharmaceutically acceptable antioxidant, preferably alpha-tocopherol or a mixture of alpha-, beta- and gamma-tocopherol.

In a fifth aspect the present disclosure relates to an emulsion according to any of the preceding aspects comprising a pharmaceutically acceptable co-surfactant, preferably oleic acid or sodium oleate.

In a sixth aspect the present disclosure relates to an emulsion according to any of the preceding aspects comprising a pharmaceutically acceptable co-solvent, preferably polyethylen glycol (PEG).

In a senventh aspect the present disclosure relates to an emulsion according to any of the preceding aspects comprising an agent for pH adjustment, preferably NaOH.

In an eighth aspect the present disclosure relates to an emulsion according to any of the preceding aspects, wherein the emulsion is an oil-in-water emulsion and wherein the oil droplets dispersed in the aqueous phase have a mean diameter of 130 to 300 nm.

In a nineth aspect the present disclosure relates to an emulsion according to any of the preceding aspects for use in anaesthesia or sedation.

In a tenth aspect the present disclosure relates to the use according to aspect 9 in patients suffering from disturbed liver function, blood coagulation disorder or inflammatory disease.

In an eleventh aspect the present disclosure relates to the use according to aspects 9 and 10, wherein the administration of the emulsion causes reduced pain on injection.

In a twelveth aspect the present disclosure relates to a method for manufacturing the emulsion according to any of the aspects 1 to 8, comprising
a) providing an oil phase comprising fish oil, fish oil extract or a mixture of fish oil, olive oil, soybean oil and medium chain triglycerides (MCT), propofol and optionally at least one pharmaceutically acceptable antioxidant and/or a pharmaceutically acceptable co-surfactant;
b) providing an aqueous phase comprising water for injection and optionally a pharmaceutically acceptable tonicity agent and/or an agent for pH adjustment and/or a pharmaceutically acceptable co-surfactant and/or a pharmaceutically acceptable co-solvent;
c) forming a pre-emulsion by mixing the oil phase provided in step a) with the aqueous phase provided in step b);
d) forming the emulsion by high-pressure homogenizing the pre-emulsion obtained in step c) and
e) sterilizing the emulsion obtained in step d),
wherein optionally either in step a) or in step b) a pharmaceutically acceptable emulsifier is added.

### Embodiments

1) Emulsion for parenteral administration comprising 0.1 to 10 wt.%, preferably 1 to 5 wt.%, propofol and 5 to 25 wt.%, preferably 10 to 20 wt.%, of an oil phase based on the total weight of the emulsion, wherein the free propofol concentration in the aqueous phase is below 0.1 % of the total propofol content and wherein the oil phase comprises fish oil, fish oil extract or a mixture of fish oil, olive oil, soybean oil and medium chain triglycerides (MCT).
2) Emulsion according to embodiment 1 comprising 10 wt.% of an oil phase comprising fish oil, fish oil extract or a mixture of fish oil, olive oil, soybean oil and medium chain triglycerides (MCT) and 1 wt.% Propofol based on the total weight of the emulsion.
3) Emulsion according to embodiment 1 comprising 10 wt.% of an oil phase comprising fish oil, fish oil extract or a mixture of fish oil, olive oil, soybean oil and medium chain triglycerides (MCT) and 2 wt.% propofol based on the total weight of the emulsion.
4) Emulsion according to embodiment 1 comprising 10 wt.% of an oil phase comprising fish oil, fish oil extract or a mixture of fish oil, olive oil, soybean oil and medium chain triglycerides (MCT) and 5 wt.% propofol based on the total weight of the emulsion.
5) Emulsion according to embodiment 1 comprising 20 wt.% of an oil phase comprising fish oil, fish oil extract or a mixture of fish oil, olive oil, soybean oil and medium chain triglycerides (MCT) and 1 wt.% propofol based on the total weight of the emulsion.
6) Emulsion according to embodiment 1 comprising 20 wt.% of an oil phase comprising fish oil, fish oil extract or a mixture of fish oil, olive oil, soybean oil and medium chain triglycerides (MCT) and 2 wt.% propofol based on the total weight of the emulsion.
7) Emulsion according to embodiment 1 comprising 20 wt.% of an oil phase comprising fish oil, fish oil extract or a mixture of fish oil, olive oil, soybean oil and medium chain triglycerides (MCT) and 5 wt.% propofol based on the total weight of the emulsion.
8) Emulsion according to any of the preceding embodiments comprising a pharmaceutically acceptable emulsifier, preferably egg lecithin or krill oil.
9) Emulsion according to any of the preceding embodiments comprising a pharmaceutically acceptable tonicity agent, preferably glycerol.
10) Emulsion according to any of the preceding embodiments comprising a pharmaceutically acceptable antioxidant, preferably alpha-tocopherol or a mixture of alpha-, beta- and gamma-tocopherol.
11) Emulsion according to any of the preceding embodiments comprising a pharmaceutically acceptable co-surfactant, preferably oleic acid or sodium oleate.
12) Emulsion according to any of the preceding embodiments comprising a pharmaceutically acceptable co-solvent, preferably polyethylen glycol (PEG).
13) Emulsion according to any of the preceding embodiments comprising an agent for pH adjustment, preferably NaOH.
14) Emulsion according to any of the preceding embodiments, wherein the emulsion is an oil-in-water emulsion and wherein the oil droplets dispersed in the aqueous phase have a mean diameter of 130 to 300 nm.
15) Emulsion according to any of the preceding embodiments for use in anaesthesia or sedation.
16) Emulsion for use according to embodiment 15 in patients suffering from disturbed liver function, blood coagulation disorder or inflammatory disease.
17) Emulsion for use according to embodiment 15 or 16, wherein the administration of the emulsion causes reduced pain on injection.
18) Method for anaesthesizing or sedating a patient comprising administering a therapeutically effective amount of an emulsion according to any of the embodiments 1 to 14.
19) Method according to embodiment 18, wherein the patient is suffering from disturbed liver function, blood coagulation disorder or inflammatory disease.
20) Method according to embodiment 18 or 19, wherein the administration of the emulsion causes reduced pain on injection.
21) Method for preparing an emulsion according to any of the embodiments 1 to 14 comprising
   a) Providing an oil phase comprising fish oil, fish oil extract or a mixture of fish oil, olive oil, soybean oil and medium chain triglycerides (MCT), propofol and optionally a pharmaceutically acceptable co-surfactant and or a pharmaceutically acceptable antioxidant,
   b) Providing an aqueous phase comprising water for injection, and optionally a pharmaceutically acceptable tonicity agent and/or an agent for pH-adjustment and/or a pharmaceutically acceptable co-solvent,
   c) Forming a pre-emulsion by mixing the oil phase obtained in step a) with the aqueous phase obtained in step b),
   d) Forming an emulsion by high pressure homogenizing the pre-emulsion obtained in step c),
   e) Sterilizing the emulsion obtained in step d),
   wherein optionally either in step a) or in step b) a pharmaceutically acceptable emulsifier is added.

### Examples

### Example 1

The emulsion was prepared from the ingredients listed in table 1.

The oil phase was prepared by mixing fish oil and Propofol. The mixture was heated to 60 °C.

The aqueous phase was prepared by mixing water, glycerol and sodium oleate. The mixture was heated to 60 °C, and then egg lecithin was added.

The pre-emulsion was formed by adding the oil phase to the aqueous phase under continous agitation using a high shear mixer (Ultra Turrax T50) at a temperature of 60 °C.

The emulsion was formed by passing the pre-emulsion six times through a Niro Soavi TwinPanda 600 homogenizer at 500 bar at a temperature of 50 °C.

The pH was adjusted to 8.0 to 9.0.

Finally the emulsion was autoclaved at 121 °C for 15 minutes.

**Table 1:**

| **Ingredient** | **Amount [g]** |
|---|---|
| Fish oil (purified, Pronova, BASF) | 10 |
| Propofol | 2 |
| Sodium oleate | 0.03 |
| Glycerol | 2.25 |
| Egg lecithin | 1.2 |
| NaOH (1 M) | to adjust pH to 8.0 - 9.0 |
| Water for injection | ad 100 |

The oil droplets of the emulsion according to example 1 had a mean diameter of 145 nm when measured directly upon sterilization using an LS 13 320 Laser Diffraction Particle Size Analyser (Beckman Coulter) according to USP <729>.

Stability data and free propofol concentration in the aqueous phase are presented in table 2.

**Table 2:**

| **Storage time [w]** | **Storage T [°C]** | **Mean droplet diameter [nm]** | **Free Propfol conc. in aq. phase [µg/ml]** |
|---|---|---|---|
| 0 | - | 145 | 2.71 |
| 1 | 25 | 146 | 2.74 |
| | 40 | 143 | 2.71 |
| 3 | 25 | 145 | 2.78 |
| | 40 | 148 | 2.77 |
| 12 | 25 | 142 | 2.72 |
| | 40 | 142 | 2.76 |
| 26 | 5 | - | 2.69 |
| | 25 | 145 | 2.89 |
| | 40 | 145 | 2.63 |

As seen in the table, neither the droplet size nor the free propofol concentration in the aqueous phase substantially changed during storage at different temperatures.

The PFAT₅ values were below 0.05 (data not shown).

Thus, the emulsion according to example 1 is stable for at least 26 weeks.

### Examples 2a and 2b

The emulsions were prepared from the ingredients listed in table 3.

The oil phases were prepared by mixing fish oil and propofol. The mixture was heated to 60 °C.

The aqueous phases were prepared by mixing water, glycerol and sodium oleate. The mixture was heated to 60 °C, and then egg lecithin was added.

The pre-emulsions were formed by adding the oil phases to the aqueous phases under continous agitation using a high shear mixer (Ultra Turrax T50) at a temperature of 60 °C.

The emulsions were formed by passing the pre-emulsions six times through a Niro Soavi TwinPanda 600 homogenizer at 500 bar at a temperature of 50 °C.

The pH was adjusted to 8.0 to 9.0.

Finally the emulsion was autoclaved at 121 °C for 15 minutes.

**Table 3:**

| **Ingredient** | **Amount [g]** | |
|---|---|---|
| | **Example 2a** | **Example 2b** |
| Fish oil (highly purified, Fresenius Kabi) | 10 | |
| Propofol | 2 | 5 |
| Sodium oleate | 0.03 | |
| Glycerol | 2.25 | |
| Egg lecithin | 1.2 | |
| NaOH (1 M) | to adjust pH to 8.0 - 9.0 | |
| Water for injection | ad 100 | |

The oil droplets of the emulsion according to example 2a had a mean diameter of 177 nm when measured directly upon sterilization. The oil doplets of the emulsion according to example 2b had a mean diameter of 155 nm when measured directly upon sterilization using an LS 13 320 Laser Diffraction Particle Size Analyser (Beckman Coulter) according to USP <729>.

Stability data and free propofol concentrations in the aqueous phase for both emulsions are presented in table 4.

As seen in the table, neither the droplet sizes nor the free propofol concentrations in the aqueous phase significantly changed during storage at different temperatues.

The PFAT₅ values were below 0.05 (data not shown).

Thus, the emulsions according to examples 2a) and 2b) are stable for at least 12 months.

**Table 4:**

| **Storage time [w]** | **Storage T [°C]** | **Mean droplet diameter [nm]** | | **Free Propofol conc. in aq. phase [µg/ml)** | |
|---|---|---|---|---|---|
| | | **2a** | **2b** | **2a** | **2b** |
| 0 | - | 177 | 155 | 2.72 | 6.07 |
| 1 | 25 | 175 | 158 | 2.95 | 6.11 |
| | 40 | 174 | 158 | 2.79 | 6.07 |
| 3 | 25 | 179 | 160 | 2.82 | 6.27 |
| | 40 | 173 | 157 | 2.79 | 6.20 |
| 12 | 25 | 180 | 167 | 2.84 | 6.62 |
| | 40 | 181 | 165 | 2.79 | 6.37 |
| 26 | 5 | - | - | 2.77 | 6.22 |
| | 25 | 165 | 159 | 3.18 | 6.40 |
| | 40 | - | 168 | 3.05 | 5.76 |
| 52 | 5 | - | - | 2.74 | 6.04 |
| | 25 | - | - | 2.59 | 6.00 |

### Example 3a, 3b, 3c and 3d

The emulsions were prepared from the ingredients listed in table 5.

The oil phases were prepared by mixing soybean oil, medium chain triglycerides, olive oil, fish oil, propofol and alpha-tocopherol. The mixture was heated to 60 °C.

The aqueous phases were prepared by mixing water, glycerol and sodium oleate. The mixture was heated to 60 °C, and then egg lecithin was added.

The pre-emulsions were formed by adding the oil phases to the aqueous phases under continous agitation using a high shear mixer (Ultra Turrax T50) at a temperature of 60 °C.

The emulsions were formed by passing the pre-emulsion six times through a Niro Soavi TwinPanda 600 homogenizer at 500 bar at a temperature of 50 °C.

The pH was adjusted to 8.0 to 9.0.

Finally the emulsions were autoclaved at 121 °C for 15 minutes.

**Table 5:**

| **Ingredient** | **Amount [g]** | | | |
|---|---|---|---|---|
| | **3a** | **3b** | **3c** | **3d** |
| Soybean oil | 3 | | 6 | |
| Medium chain triglycerides | 3 | | 6 | |
| Olive oil | 2.5 | | 5 | |
| Fish oil | 1.5 | | 3 | |
| Propofol | 2 | 5 | 2 | 5 |
| Alpha-tocopherol | 0.02 | | | |
| Sodium oleate | 0.03 | | | |
| Glycerol | 2.25 | | | |
| NaOH (1 M) | to adjust pH to 8.0 - 9.0 | | | |
| Water for injection | ad 100 | | | |

The oil droplets of the emulsion according to example 3 had a mean diameter of 163 nm (3a), 149 nm (3b), 289 nm (3c) and 181 nm (3d) respectively when measured directly upon sterilization using an LS 13 320 Laser Diffraction Particle Size Analyser (Beckman Coulter) according to USP <729>.

Stability data and free propofol concentrations in the aqueous phase for these four emulsions are presented in table 6.

As seen in the table, neither the droplet size nor the free propofol concentrations in the aqueous phase significantly changed during storage at different temperatures.

The PFAT₅ values were below 0.05 (data not shown).

Thus, the emulsions according to examples 3a, 3b, 3c and 3d are stable for at least 12 months.

**Table 6:**

| **Storage time [w]** | **Storage T [°C]** | **Mean droplet diameter [nm]** | | | | **Free Propfol conc. in aq. Phase [µg/ml]** | | | |
|---|---|---|---|---|---|---|---|---|---|
| | | **3a** | **3b** | **3c** | **3d** | **3a** | **3b** | **3c** | **3d** |
| 0 | - | 163 | 149 | 289 | 181 | 2.18 | 5.16 | 1.12 | 2.82 |
| 1 | 25 | - | - | - | - | 2.22 | 5.46 | 1.13 | 2.84 |
| | 40 | - | - | - | - | 2.24 | 5.23 | 1.14 | 2.92 |
| 3 | 25 | 152 | 146 | 257 | 198 | 2.21 | 5.20 | 1.16 | 3.66 |
| | 40 | 166 | 164 | 276 | 217 | 2.22 | 5.27 | 1.14 | 2.89 |
| 12 | 25 | 160 | 148 | 273 | 191 | 2.25 | 5.53 | 1.02 | 2.89 |
| | 40 | 156 | 159 | 294 | 191 | 2.19 | 5.45 | 1.02 | 2.90 |
| 26 | 5 | - | - | - | - | 2.53 | 6.17 | 1.48 | 2.79 |
| | 25 | 153 | 146 | 285 | 184 | 2.65 | 5.65 | 1.11 | 3.02 |
| | 40 | 156 | 147 | 278 | 187 | 2.14 | 4.89 | 1.10 | 2.70 |
| 52 | 5 | - | - | - | - | 2.20 | 5.15 | 1.17 | 2.99 |
| | 25 | - | - | - | - | 2.22 | 5.09 | 1.12 | 2.79 |

## Claims

1. Emulsion for parenteral administration comprising 0.1 to 10 wt.%, preferably 1 to 5 wt.%, propofol and 5 to 25 wt.%, preferably 10 to 20 wt.%, of an oil phase based on the total weight of the emulsion, wherein the free propofol concentration in the aqueous phase is below 0.1 % of the total propofol content and wherein the oil phase comprises fish oil, fish oil extract or a mixture of fish oil, olive oil, soybean oil and medium chain triglycerides (MCT).

2. Emulsion according to claim 1 comprising a pharmaceutically acceptable emulsifier, preferably egg lecithin or krill oil.

3. Emulsion according to claim 1 or 2 comprising a pharmaceutically acceptable tonicity agent, preferably glycerol.

4. Emulsion according to any of the preceding claims comprising a pharmaceutically acceptable antioxidant, preferably alpha-tocopherol or a mixture of alpha-, beta- and gamma-tocopherol.

5. Emulsion according to any of the preceding claims comprising a pharmaceutically acceptable co-surfactant, preferably oleic acid or sodium oleate.

6. Emulsion according to any of the preceding claims comprising a pharmaceutically acceptable co-solvent, preferably polyethylen glycol (PEG).

7. Emulsion according to any of the preceding claims comprising an agent for pH adjustment, preferably NaOH.

8. Emulsion according to any of the preceding claims, wherein the emulsion is an oil-in-water emulsion and wherein the oil droplets dispersed in the aqueous phase have a mean diameter of 130 to 300 nm.

9. Emulsion according to any of the preceding claims for use in anaesthesia or sedation.

10. Emulsion for use according to claim 9 in patients suffering from disturbed liver function, blood coagulation disorder or inflammatory disease.

11. Emulsion for use according to claim 9 or 10, wherein the administration of the emulsion causes reduced pain on injection.

12. Method for preparing an emulsion according to any of the claims 1 to 8 comprising
a) Providing an oil phase comprising fish oil, fish oil exract or a mixture of fish oil, olive oil, soybean oil and MCT, propofol and optionally a pharmaceutically acceptable co-surfactant and or a pharmaceutically acceptable antioxidant,
b) Providing an aqueous phase comprising water for injection, and optionally a pharmaceutically acceptable tonicity agent and/or an agent for pH-adjustment and/or a pharmaceutically acceptable co-solvent,
c) Forming a pre-emulsion by mixing the oil phase obtained in step a) with the aqueous phase obtained in step b),
d) Forming an emulsion by high pressure homogenizing the pre-emulsion obtained in step c),
e) Sterilizing the emulsion obtained in step d),
wherein optionally either in step a) or in step b) a pharmaceutically acceptable emulsifier is added.

## Patentansprüche

1. Emulsion zur parenteralen Verabreichung umfassend 0,1 bis 10 Gew.-%, vorzugsweise 1 bis 5 Gew.-%, Propofol und 5 bis 25 Gew.-%, vorzugsweise 10 bis 20 Gew.-%, einer Ölphase basierend auf dem Gesamtgewicht der Emulsion, wobei die freie Propofolkonzentration in der wässrigen Phase unter 0,1% des gesamten Propofolgehaltes liegt und wobei die Ölphase Fischöl, Fischölextrakt oder eine Mischung aus Fischöl, Olivenöl, Sojabohnenöl und mittelkettigen Triglyceriden (MCT) umfasst.

2. Emulsion nach Anspruch 1, umfassend einen pharmazeutisch akzeptablen Emulgator, vorzugsweise EiLecithin oder Krillöl.

3. Emulsion nach Anspruch 1 oder 2, umfassend ein pharmazeutisch akzeptables Tonizitätsmittel, vorzugsweise Glycerin.

4. Emulsion nach einem der vorhergehenden Ansprüche, umfassend ein pharmazeutisch akzeptables Antioxidans, vorzugsweise Alpha-Tocopherol oder eine Mischung aus Alpha-, Beta- und Gamma-Tocopherol.

5. Emulsion nach einem der vorhergehenden Ansprüche, umfassend ein pharmazeutisch akzeptables Co-Tensid, vorzugsweise Ölsäure oder Natriumoleat.

6. Emulsion nach einem der vorhergehenden Ansprüche, umfassend ein pharmazeutisch akzeptables Co-Solvens, vorzugsweise Polyethylenglycol (PEG).

7. Emulsion nach einem der vorhergehenden Ansprüche, umfassend ein Mittel zur Einstellung des pH-Wertes, vorzugsweise NaOH.

8. Emulsion nach einem der vorhergehenden Ansprüche, wobei die Emulsion eine Öl-in-Wasser-Emulsion ist und wobei die in der wässrigen Phase dispergierten Öltröpfchen einen mittleren Durchmesser von 130 bis 300 nm aufweisen.

9. Emulsion nach einem der vorhergehenden Ansprüche, für deren Verwendung bei Betäubung oder Sedierung.

10. Emulsion für deren Verwendung nach Anspruch 9 in Patienten, welche unter veränderter Leberfunktion, Blutgerinnungsstörung oder entzündlicher Erkrankung leiden.

11. Emulsion für deren Verwendung nach Anspruch 9 oder 10, wobei die Verabreichung der Emulsion Schmerzlinderung bei Injektion verursacht.

12. Verfahren zur Zubereitung einer Emulsion nach einem der Ansprüche 1 bis 8, umfassend
a) das Bereitstellen einer Ölphase umfassend Fischöl, Fischölextrakt oder eine Mischung aus Fischöl, Olivenöl, Sojabohnenöl und MCT, Propofol und wahlweise ein pharmazeutisch akzeptables Co-Tensid und/oder ein pharmazeutisch akzeptables Antioxidans,
b) das Bereitstellen einer wässrigen Phase umfassend Wasser für Injektionszwecke, und wahlweise ein pharmazeutisch akzeptables Tonizitätsmittel und/oder ein Mittel zur Einstellung des pH-Wertes und/oder ein pharmazeutisch akzeptables Co-Solvens,
c) das Bilden einer Voremulsion mittels des Mischens der in Schritt a) erhaltenen Ölphase mit der in Schritt b) erhaltenen wässrigen Phase,
d) das Bilden einer Emulsion mittels der Hochdruckhomogenisierung der in Schritt e) erhaltenen Voremulsion,
e) das Sterilisieren der in Schritt d) erhaltenen Emulsion,
wobei wahlweise entweder in Schritt a) oder in Schritt b) ein pharmazeutisch akzeptabler Emulgator hinzugefügt wird.

## Revendications

1. Emulsion pour administration parentérale comprenant 0,1 à 10 % en poids, de préférence 1 à 5 % en poids de propofol et 5 à 25 % en poids, de préférence 10 à 20 % en poids d'une phase huileuse basée sur le poids total de l'émulsion, dans laquelle la concentration libre de propofol dans la phase aqueuse est inférieure à 0,1 % du contenu total de propofol et dans laquelle la phase huileuse comprend de l'huile de poisson, de l'extrait d'huile de poisson ou un mélange d'huile de poisson, de l'huile d'olive, de l'huile de soja et des triglycérides à chaîne moyenne (TCM).

2. Emulsion selon la revendication 1 comprenant un émulsifiant pharmaceutiquement acceptable, de préférence de la lécithine d'œuf ou de l'huile de krill.

3. Emulsion selon la revendication 1 ou 2 comprenant un agent de tonicité pharmaceutiquement acceptable, de préférence du glycérol.

4. Emulsion selon l'une quelconque des revendications précédentes comprenant un antioxydant pharmaceutiquement acceptable, de préférence de l'alpha-tocophérol ou un mélange d'alpha-, bêta- et gamma-tocophérol.

5. Emulsion selon l'une quelconque des revendications précédentes comprenant un co-surfactant pharmaceutiquement acceptable, de préférence de l'acide oléique ou de l'oléate de sodium.

6. Emulsion selon l'une quelconque des revendications précédentes comprenant un co-solvant pharmaceutiquement acceptable, de préférence polyéthylène glycol (PEG).

7. Emulsion selon l'une quelconque des revendications précédentes comprenant un agent pour l'ajustement du pH, de préférence du NaOH.

8. Emulsion selon l'une quelconque des revendications précédentes, dans laquelle l'émulsion est une émulsion huile dans l'eau et dans laquelle les gouttes d'huile dispersées dans la phase aqueuse ont un diamètre moyen de 130 à 300 nm.

9. Emulsion selon l'une quelconque des revendications précédentes à utiliser dans l'anesthésie ou la sédation.

10. Emulsion à utiliser selon la revendication 9 chez des patients atteints de fonction hépatique perturbée, trouble de la coagulation du sang ou d'une maladie inflammatoire.

11. Emulsion à utiliser selon la revendication 9 ou 10, dans laquelle l'administration de l'émulsion produit une réduction de la douleur au moment de l'injection.

12. Procédé de préparation d'une émulsion selon l'une quelconque des revendications 1 à 8 comprenant
a) la fourniture d'une phase huileuse comprenant de l'huile de poisson, un extrait d'huile de poisson ou un mélange d'huile de poisson, de l'huile d'olive, de l'huile de soja et des TCM, du propofol et en option un co-surfactant pharmaceutiquement acceptable et/ou un antioxydant pharmaceutiquement acceptable,
b) la fourniture d'une phase aqueuse comprenant de l'eau pour injection, et en option un agent de tonicité pharmaceutiquement acceptable et/ou un agent d'ajustement de pH et/ou un co-solvant pharmaceutiquement acceptable,
c) la formation d'une pré-émulsion en mélangeant la phase huileuse obtenue dans l'étape a) avec la phase aqueuse obtenue dans l'étape b),
d) la formation d'une émulsion par homogénéisation à haute pression de l'émulsion obtenue dans l'étape e),
e) la stérilisation de l'émulsion obtenue dans l'étape d),
dans lequel optionnellement, soit dans l'étape a) ou dans l'étape b) un émulsifiant pharmaceutiquement acceptable est ajouté.
